# EUROPÄISCHE PATENTSCHRIFT

(11) **EP 1 165 753 B1**
(45) Veröffentlichungstag und Bekanntmachung des Hinweises auf die Patenterteilung: **07.12.2005**
(21) Anmeldenummer: 00920340.7
(22) Anmeldetag: 18.02.2000
(51) Int. Cl.: C12N 5/08, C12M 3/04, G01N 1/06

(54) **VERFAHREN ZUR KULTIVIERUNG VON KREBSZELLEN AUS HUMANGEWEBE UND VORRICHTUNG ZUR AUFBEREITUNG VON GEWEBEPROBEN**
METHOD FOR CULTIVATING CANCER CELLS FROM HUMAN TISSUE AND DEVICE FOR PREPARING TISSUE SAMPLES
PROCEDE DE CULTURE DE CELLULES CANCEREUSES TIREES DE TISSUS HUMAINS ET DISPOSITIF DE PREPARATION D'ECHANTILLONS DE TISSUS

(30) Priorität: 10.03.1999 DE 19912798
(43) Veröffentlichungstag der Anmeldung: 02.01.2002
(73) Patentinhaber: Magforce Applications GmbH, 14050 Berlin (DE)
(72) Erfinder: JORDAN, Andreas, 14167 Berlin (DE)
(74) Vertreter: Arth, Hans-Lothar
(86) Internationale Anmeldenummer: PCT/DE2000/000528
(87) Internationale Veröffentlichungsnummer: WO 2000/053728

(56) Entgegenhaltungen:
- WO-A-95/24464
- WO-A-97/23602
- DE-A- 4 334 281
- DE-C- 19 912 798
- US-A- 5 985 290
- IMAGAWA W ET AL: "SERUM-FREE GROWTH OF NORMAL AND TUMOR MOUSE MAMMARY EPITHELIAL CELLS IN PRIMARY CULTURE" PROCEEDINGS OF THE NATIONAL ACADEMY OF SCIENCES OF THE UNITED STATES, Bd. 79, Nr. 13, 1982, Seiten 4074-4077, XP000965632 1982 ISSN: 0027-8424
- PATENT ABSTRACTS OF JAPAN vol. 1995, no. 11, 26. Dezember 1995 (1995-12-26) & JP 07 218398 A (SEITAI KAGAKU KENKYUSHO:KK), 18. August 1995 (1995-08-18)

## Beschreibung

Die Erfindung betrifft ein Verfahren zur Kultivierung von Krebszellen aus Humangewebe für naturwissenschaftliche, vorzugsweise molekularbiologische und zellbiologische Reihenuntersuchungen sowie ein Zellkulturmedium zur Durchführung des Verfahrens und eine Vorrichtung zur Aufarbeitung der Gewebeproben.

Für molekularbiologische Untersuchungen, vor allem mit prädiktiver Intention, ist die Kultur von primären, aus Feinnadel- und Stanzbiopsien gewonnenem Zellmaterial von zentraler Bedeutung. Bei den bekannten Methoden ist jedoch die "Angehrate" der aus Humangewebe isolierten Zellen sehr gering. Bei manchen Tumorarten ist eine Zellkultivierung bisher nicht gelungen. Diese Problematik ist zum einen dadurch begründet, daß die das Tumorgewebe umgebenden Normalzellen und/oder die das Tumorgewebe infiltrierenden Bindegewebszellen als erste wachsen und so die Tumorzellen, die für die Untersuchungen gewonnen werden sollen, überwachsen und am Wachstum hindern. Eine erfolgreiche Kultivierung von Krebszellen wird darüber hinaus durch vielfältige Kontaminationen, insbesondere mit Bakterien oder Pilzen, verhindert.

Die bisher für die Kultivierung von Tumorzellen verwendeten Zellkulturmedien, wie RPMI 1640, Basal Medium Eagle, ISCOVE's, Medium 199, Leibovitz L-15. u.a., sind nicht in der Lage, das Wachstum der Krebszellen in ausreichendem Maße zu fördern bzw. das der Normalzellen und bakteriellen Kontaminanten zu verhindern. Der bisher übliche unkontrollierte Einsatz von Antibiotika wirkt zwar den negativen Einflüssen der Kontaminanten entgegen, behindert aber gleichzeitig das Wachstum der Tumorzellen.

Bei den bekannten Verfahren zur Kultivierung von Krebszellen erfolgt die Aufbereitung der aus Feinnadel- oder Stanzbiopsien gewonnenen Gewebeproben durch eine mechanisch-enzymatische Gewebe-Disaggregation, bei der der heterogene, aus verschiedenen Schichten bestehende, die Gewebeprobe bildende Stanzzylinder durch eine Feinzerkleinerung als Ganzes in eine breiige Masse zerteilt und mit Hilfe von Enzymen in einzelne Zellen umgewandelt werden soll. Durch diese Feinzerkleinerung wird aber die heterogene Struktur der entnommenen Gewebeprobe zerstört und es erfolgt eine intensive Vermischung der Tumorzellen mit den Normalzellen und Kontaminanten. Zum einen wird die Vitalität der Tumorzellen durch die Feinzerkleinerung beeinträchtigt. Andererseits ist durch die Zerstörung der Heterogenität der Gewebeprobe der Probematerialbrei mit Normalzellen und Kontaminanten durchsetzt, so daß das Wachstum der Tumorzellen aus den oben genannten Gründen vermindert oder sogar vollständig verhindert wird. Bei dieser Art der mechanisch-enzymatischen Gewebe-Desaggregation des Gesamtmaterials sind Aussagen über die Struktur des Tumors nicht möglich.

Bei den bisher bekannten Verfahren zur Vermehrung und Reinigung des aus einer Gewebeprobe gewonnenen Tumormaterials wird die Zellvermehrung durch Transplantation des Tumormaterials auf eine Nacktmaus vorgenommen (Xeno-Transplantat). Bei diesem Verfahren können zwar Angehraten des transplantierten Tumorgewebes von 50 % und gelegentlich auch mehr erreicht werden, jedoch vergehen - je nach Tumorart und Eigenschaften - mehrere Wochen oder gar Monate, ehe sich eine Zellvermehrung in vivo einstellt. Aufgrund des erheblichen Aufwands und der Zeitdauer haben Routinetests mit patientenindividuellen Primärzellen für eine Verlaufskontrolle und prädiktive Zwecke bisher nicht Eingang in die klinische Anwendung gefunden. Aufgrund der langwierigen und oftmals vergeblichen Versuche zur Kultivierung des vom Patienten entnommenen Gewebematerials liegen die Testergebnisse viel zu spät vor, um in einem Routineverfahren aus den Versuchsergebnisse eine rechtzeitige Änderung des Therapieregimes vornehmen zu können. Neben dem hohen Zeitaufwand ist auch die erforderliche Durchführung von Tierversuchen und der hohe Kostenaufwand nachteilig.

Der Erfindung liegt daher die Aufgabe zugrunde, ein von Tierversuchen unabhängiges Verfahren zur Kultivierung von Krebszellen aus Humangewebeproben anzugeben, das in einem vergleichsweise kurzen Zeitraum von wenigen Tagen eine sichere Vermehrung von Tumorzellen aus einer Gewebeprobe gewährleistet und reproduzierbare Aussagen über die Struktur und die Malignität des Tumors sowie Wachstums- und Strukturänderungen oder therapeutische Effekte zuläßt. Ein weiteres Ziel der Erfindung besteht in der Bereitstellung einer auf dem Verfahren beruhenden Vorrichtung zur reproduzierbaren Aufbereitung von Gewebeproben sowie einem geeigneten Zellkulturmedium zur Vermehrung von Krebszellen in vitro.

Erfindungsgemäß wird die Aufgabe mit einem Verfahren zur Kultivierung von Krebszellen gemäß den Merkmalen des Patentanspruchs I gelöst.

Der Grundgedanke der Erfindung besteht dabei darin, daß die Gewebeprobe in einem sequentiell-parallelen Schneidvorgang in eine Mehrzahl separater Gewebesegmente aufgeteilt wird und somit die auf der Grundlage von Kontaminanten, Normalzellen und Tumorzellen heterogene Gewebeprobe bzw. deren Heterogenität örtlich aufgelöst wird. Die Gewebesegmente werden dann jeweils separat weiter zerkleinert und die so gebildeten kleinen, separierten Gewebefragmente und -flüssigkeiten sowie die bei der sequentiell-parallelen Teilung der Gewebeprobe entstandene, ebenfalls jeweils separat gewonnene Gewebeflüssigkeit werden in einem spezifischen Zellkulturmedium und unter ausgewählten Kulturbedingungen kultiviert. Durch die örtliche Auflösung der Gewebeprobe werden die Einflüsse von in dieser vorhandenen Normalzellen, die bei üblicher - mechanischer oder enzymatischer - Aufbereitung der Gewebeprobe ein Überwachsen der Tumorzellen bewirken, ausgeschaltet oder zurückgedrängt. Gleichermaßen wird das Ausmaß von Kontaminationen, wie Pilze oder Bakterien, wesentlich reduziert bzw. erforderliche Antibiotika, die die Vermehrung von Tumorzellen bekanntermaßen stören, können sparsam und gezielt eingesetzt werden, ohne sich auf die Krebszellenkultivierung negativ auszuwirken.

Das vorgeschlagene Verfahren wird in weiterer Ausbildung der Erfindung durch ein für sehr geringe Gewebemengen geeignetes Zellkulturmedium in der im Anspruch 8 wiedergegebenen Zusammensetzung sowie durch die im Anspruch 5 hinsichtlich der Sauerstoff- und Kohlendioxidatmosphäre, der Luftfeuchte und der Temperatur sowie der Verwendung einer Biomatrix dargelegten Kultivierungsbedingungen vorteilhaft ergänzt. Insgesamt werden somit Bedingungen geschaffen, unter denen das Wachstum der Krebszellen gefördert und das der Normalzellen und Kontaminanten wesentlich eingeschränkt oder sogar vollständig ausgeschaltet wird.

Weitere vorteilhafte Verfahrensmerkmale ergeben sich aus den Unteransprüchen. So wurde beispielsweise gefunden, daß die Tumorzellen in Anwesenheit von Erythrozyten, die unmittelbar von der Entnahmestelle der Gewebeprobe am Patienten stammen, besonders schnell wachsen und eine höhere "Angehrate" besitzen als Zellen, die nur im reinem Zellkulturmedium zum Wachstum gebracht werden. Darüber hinaus hat es sich als vorteilhaft erwiesen, wenn die Gewebeprobe mindestens 2 Stunden, aber nicht länger als 24 Stunden, bei etwa 4°C bis 12°C in einem Zellkulturmedium aufbewahrt wird, um bereits jetzt an das Zellkulturmedium, in dem später die Vermehrung der Krebszellen erfolgen soll, angepaßt zu werden. Unter den aufgeführten Verfahrensbedingungen ist eine Adhäsion der Tumorzellen aus den aufbereiteten Gewebefragmenten an einer Biomatrix in der Zellkulturflasche bereits nach 1 bis 12 Stunden zu verzeichnen, sofern eine dem Gewebeentnahmeart identische Kulturtemperatur eingestellt wird.

Mit der vorliegenden und weiter unten in einem Ausführungsbeipiel detailliert beschriebenen Erfindung wird somit ein Verfahren zur Vermehrung von Tumorzellen in vitro angegeben, mit dem in einem vergleichsweise kurzen Zeitraum bei allen Tumorarten eine Kultivierung von aus Humangewebe gewonnenen Zellen mit einer "Angehrate" von 100 % gelingt. Gegenüber der auf der Nacktmaus (Xeno-Transplantat) erzielten Zellvermehrung, bei der Angehraten von etwa 50 % erzielt werden, werden nicht nur Tierversuche vermieden und Kosten gespart, sondern es ist auch eine drastische Verkürzung der für die Vermehrung des Zellmaterials benötigten Zeit zu verzeichnen, die gegenüber einem Zeitraum von mehreren Wochen oder gar Monaten beim Tumorwachstum in vivo bei dem erfindungsgemäß vorgeschlagenen Verfahren in der Regel bei lediglich 1 - 10 Tagen liegt.

Dadurch ist es erstmals möglich, Routineuntersuchungen für die Verlaufskontrolle von Krebserkrankungen und für prädiktive Zwecke (Sensibilitätstest mit Strahlen, Chemotherapie, Hyperthermie) oder zur Früherkennung von Resistenzen zur Identifikation neuer Antikrebswirkstoffe, als Ergänzung für zytologische oder histopathologische Gewebebefunde und in der Grundlagenforschung auf einfache und reproduzierbare Weise schnell und kostengünstig durchzuführen.

Nach einem weiteren Merkmal der Erfindung ist zur erfindungsgemäß ortsaufgelösten Aufbereitung der Gewebeprobe als entscheidende Voraussetzung für die erfolgreiche Zellvermehrung eine Vorrichtung gemäß den Merkmalen des Anspruchs 9 vorgesehen. Diese Vorrichtung besteht zum einen aus einem Schneidapparat zum sequentiell-parallelen Teilen der Gewebeprobe und zum anderen aus einem Zerkleinerungsgerät zur weiteren Aufarbeitung der mit dem Schneidapparat hergestellten Gewebesegmente. Bei den in diesen Vorrichtungen durchgeführten Schneidvorgängen werden die jeweiligen Gewebestücke und -flüssigkeiten, die von verschiedenen Stellen der heterogenen Gewebeprobe stammen, voneinander getrennt in der Vorrichtung gehalten und können somit selektiv für die Zellvermehrung eingesetzt werden.

Der Schneidapparat umfaßt im wesentlichen eine in Kammern eingeteilte Auffangschale mit einer auf dieser beweglich angebrachten Schneidplatte sowie einen Schneidmesserrahmen. In der Schneidplatte in vorgegebenen Abständen ausgebildete Schneidrinnen, die im Schneidbereich zur Auffangschale hin offen sind, liegen jeweils oberhalb einer Kammer. Im Schneidmesserrahmen sind Schneidmesser oder Schneiddrähte in einem Abstand angebracht, der mit dem zwischen den Schneidrinnen übereinstimmt. Durch das Schneiden der auf der Schneidplatte liegenden Gewebeprobe jeweils im Bereich einer Schneidrinne wird ein sauberes Abtrennen der Gewebesegmente erreicht, und gleichzeitig gelangen Reststücke und Gewebeflüssigkeit in die unterhalb der Schneidrinne liegende Kammer.

Zur weiteren Aufbereitung der Gewebesegmente ist ein Zerkleinerungsgerät vorgesehen, das aus einer in Kammern geteilten Flüssigkeits-Auffangschale, einer auf dieser lösbar angebrachten Aufbereitungsplatte mit Vertiefungen zur Aufnahme der Gewebesegmente und einzelnen oder in einer Halteplatte drehbar und längsbeweglich angeordneten Drehstempeln mit an deren Stirnseite befestigten Messern besteht. Mit diesem Gerät werden die zur Zellvermehrung letztlich eingesetzten kleinen Gewebefragmente erzeugt. Die gleichfalls verwendbare, bei dem Schneidvorgang entstehende Gewebeflüssigkeit gelangt über in den Vertiefungen ausgebildete Löcher in die jeweils darunterliegende Kammer in der Flüssigkeits-Auffangschale und wird ebenfalls zur Zellvermehrung verwendet.

Weitere Merkmale und vorteilhafte Weiterbildungen der erfindungsgemäßen Vorrichtung ergeben sich aus den übrigen Unteransprüchen.

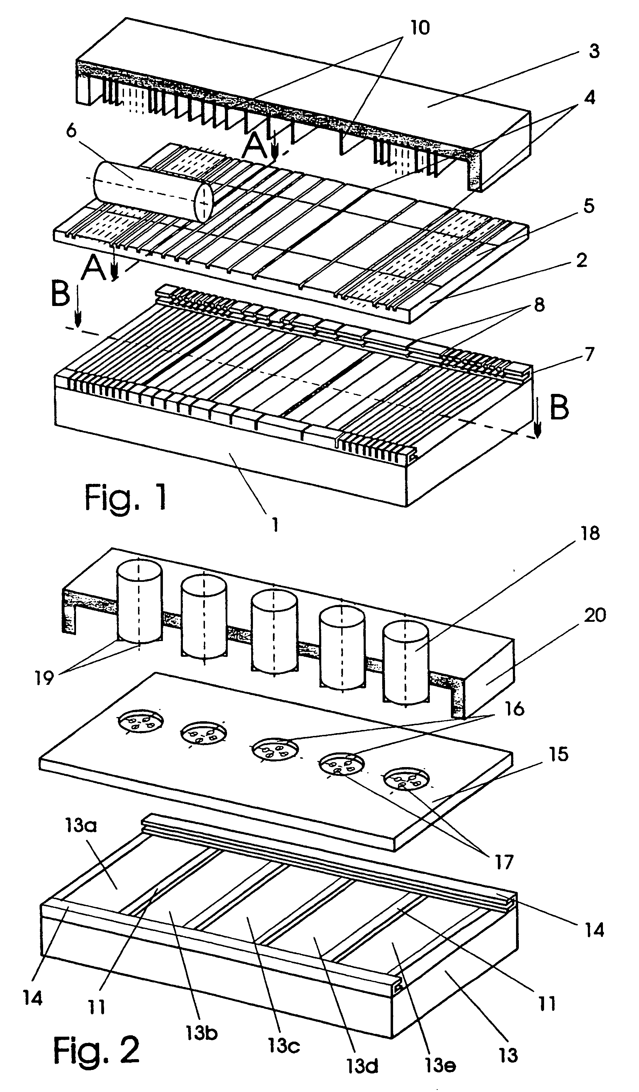

Ein Ausführungsbeispiel der Erfindung wird nachfolgend beschrieben, wobei hinsichtlich der Zellvermehrung in vitro auf die beigefügte Tabelle, in der die Zusammensetzung des verwendeten Zellkulturmediums wiedergegeben ist, und hinsichtlich der Aufarbeitung der Gewebeproben auf die beigefügte Zeichnung Bezug genommen wird. In der Zeichnung zeigen:
Fig . 1
   eine auseinandergezogene perspektivische Ansicht einer Schneidvorrichtung zur erfindungsgemäßen sequentiell-parallelen Aufbereitung einer Gewebeprobe;
Fig. 2
   eine auseinandergezogene perspektivische Darstellung einer Vorrichtung zur weiteren Aufbereitung der in der Vorrichtung nach Fig. 1 hergestellten Gewebesegmente für die Zellvermehrung in vitro;
Fig. 3
   eine Schnittansicht einer Auffangschale längs der Linie B-B in Fig. 1;
Fig. 4
   einen senkrechten Schnitt durch eine Schneidplatte im Bereich einer Schneidrinne längs der Linie A-A in Figur 1; und
Fig. 5
   eine Querschnittsansicht der Vorrichtung nach Fig. 2 in zusammengebautem Zustand.

Die vom Patienten in Form einer Feinnadel-bzw. Stanz-Biopsie entnommene Gewebeprobe liegt als Gewebestanzzylinder vor, kann aber auch ein nach anderen Verfahren gewonnenes kleines Gewebefragment oder ein Gewebestück unterschiedlicher Form und Größe sein. Die Gewebeprobe mit den an dieser haftenden, von der Entnahmestelle der Probe am Patienten stammenden Erythrozyten wird für den Transport zum Untersuchungsort in einem Zellkulturmedium bei Temperaturen zwischen 2°C und 12°C aufbewahrt, und zwar für einen Zeitraum von mindestens 2 Stunden, maximal jedoch 24 Stunden. In diesem Zeitraum werden mechanische Belastungen des Gewebestücks vermieden. Das Gewebestück kann sich dabei bereits an das auch zur Zellvermehrung später benutzte Zellkulturmedium mit gleicher Zusammensetzung anpassen, wobei die oben angegebenen Temperaturen besonders günstig sind.

Die Aufarbeitung der Gewebeprobe für die Zellkultivierung erfolgt mit den in der Zeichnung dargestellten Vorrichtungen.

Die Vorrichtung zum sequentiell-parallelen Teilen der Gewebeprobe in Abschnitte von bestimmter, vorgegebener Länge und zum Separieren dieser Abschnitte bzw. von Bestandteilen derselben umfaßt eine Auffangschale 1, eine auf der Auffangschale 1 gehaltene Schneidplatte 2 und einen Schneidmesserrahmen 3. Die Schneidplatte 2 ist in fünf Abschnitte gleicher Breite eingeteilt. In jedem Abschnitt befinden sich jeweils in unterschiedlichem Abstand angeordnete Schneidrinnen 4, die in ihrem mittleren Bereich zur Auffangsschale 1 hin offen sind. Die Schneidrinnen 4 sind in den fünf Abschnitten jeweils im Abstand von 1 mm, 2 mm, 3 mm, 5 mm und 1 mm angeordnet. In Längsrichtung der Schneidplatte ist in deren mittlerem Bereich eine aufgerauhte Auflagefläche 5 ausgebildet, um die darauf liegende Gewebeprobe 6 bei einem Schneidvorgang zu fixieren. In diesem Bereich sind die Schneidrinnen 4 nach unten offen. Die Schneidplatte 2 ist in zwei an den Längsseiten der Auffangschale 1 angebrachten Führungsschienen 7 verschiebbar gehalten. Die Schneidrinnen 4 in der Schneidplatte 2 setzen sich in Einschnitten 8 in den Führungsschienen fort.

Die Auffangschale 1 ist entsprechend den in der Schneidplatte 2 vorgesehenen Rinnenabschnitten durch Trennwände 12 in fünf Kammern 1a - 1e geteilt, wobei den Schneidrinnen durch weitere Zwischenwände in den betreffenden Kammern 1a, 1b, 1c und 1e jeweils Unterkammern 1a1 bis 1a9, 1b1 bis 1b4, 1c1 bis 1c3 und 1e1 bis 1e9 zugeordnet sind. Die Unterkammern sind zur exakten örtlichen Zuordnung der einzelnen Gewebeprobesegmente 6a oder Gewebeprobenreste bzw. Gewebeflüssigkeit entsprechend unterschiedlich markiert.

Der Schneidmesserrahmen 3 besteht aus einem Deckel oder Halterahmen mit an dessen Deckplatte befestigten Schneidmessern 10. Anstelle der Schneidmesser 10 können zwischen den Rahmenseiten auch Schneiddrähte gespannt sein. Die Schneidmesser 10 sind im gleichen Abstand wie die Schneidrinnen 4 in der Schneidplatte angeordnet. Der Schneidmesserrahmen 3 ist so dimensioniert bzw. die Schneidmesser 10 sind so angeordnet, daß die Schneidelemente über bzw. in den Schneidrinnen 4 und Einschnitten 8 hin- und herbewegt werden können. Der Schneidmesserrahmen 3 kann an einer Längsseite der Auffangschale 1 gelenkig befestigt sein, und zwar so, daß er in der auf der Schneidplatte 2 befindlichen Lage dennoch quer zur Gewebeprobe bewegt werden kann, um die Probe an den Schnittstellen sauber zu durchtrennen.

In Abhängigkeit von der Länge der Gewebeprobe und dem gewünschten Schnittabstand wird die Gewebeprobe 6 auf die aufgerauhte Auflage 5 der Schneidplatte 2 gelegt. Durch Hin- und Herbewegen des auf die Gewebeprobe gelegten (geklappten) Schneidmesserrahmens 3 wird das Präparat im Bereich der Schneidrinnen 4 durchtrennt. Beim Schneiden entstehende Flüssigkeit fließt über die im Bereich der Auflagefläche 5 der Schneidplatte 2 in den Schneidrinnen 4 vorgesehenen Öffnungen 4a in die jeweils darunter liegende Unterkammer. Die aufgefangene Flüssigkeit kann ebenso für die Zellvermehrung genutzt werden wie die abgetrennten Probensegmente, die entweder auf der Schneidplatte 2 liegenbleiben oder durch die Öffnung 4a in der Schneidrinne 4 in die darunterliegende Unterkammer fallen.

Mit der beschriebenen Vorrichtung gemäß den Figuren 1,3 und 4 können in den vorgegebenen Abmessungen präzise und gleichmäßige sowie glatte Schnitte ohne Beschädigung des Probenmaterials reproduzierbar ausgeführt werden. Die Zellvermehrung erfolgt somit aus einer entsprechend der Heterogenität des dem Patienten entnommenen Gewebestücks durch die Segmentierung örtlich aufgelösten Gewebeprobe. Dadurch wird der störende Einfluß von Normalzellen und Kontaminanten auf das Wachstum der Tumorzellen zurückgedrängt bzw. ausgeschaltet (Selektion). Außerdem kann auch die beim Schneiden der Gewebeprobe ortsaufgelöst entstehende Gewebeflüssigkeit, die wichtige Stammzellen enthält, für die Zellvermehrung genutzt werden. Im Ergebnis der sich an die unten beschriebene weitere Aufbereitung der Gewebeprobe anschließenden Zellvermehrung in vitro können Aussagen über die Struktur der heterogen ausgebildeten Gewebeprobe, über die Anordnung des Tumorkerns oder die Malignität getroffen werden. Schließlich ist die Herstellung der Probensegmente mit der jeweiligen Ortsauflösung auch reproduzierbar, so daß verläßliche Aussagen über den Verlauf der Erkrankung oder die Wirkung therapeutischer Maßnahmen getroffen werden können.

In einer Ausführungsvariante der oben beschriebenen Schneidvorrichtung kann das Durchtrennen der Gewebeprobe auch mit einem separaten Messerstempel (nicht dargestellt) erfolgen, an dem die Schneidmesser oder Schneiddrähte in einem Abstand angebracht sind, der mit dem zwischen den Schneidrinnen 4 übereinstimmt.

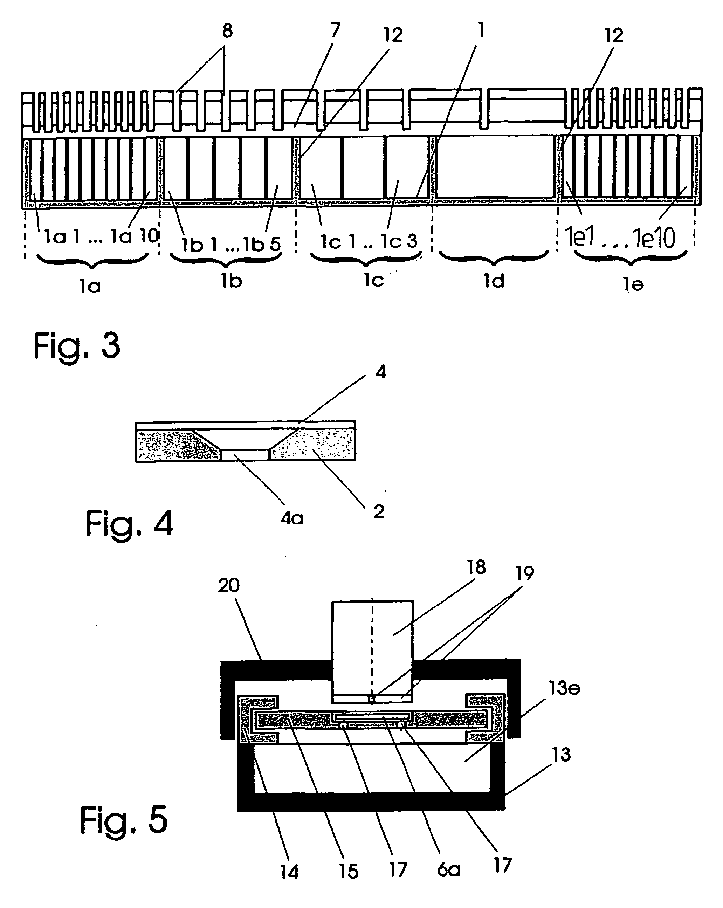

In den Figuren 2 und 5 ist eine Vorichtung zur weiteren Aufbereitung der ortsaufgelöst zur Verfügung gestellten Probensegmente 6a oder von Reststücken wiedergegeben. Bei dieser Vorrichtung ist eine Flüssigkeits-Auffangschale 13 durch senkrechte Trennwände 11 in mehrere Kammern 13a bis 13e aufgeteilt. In zwei am oberen Rand der Flüssigkeits-Auffangschale 13 gegenüberliegend angebrachten Führungsschienen 14 ist eine Aufbereitungsplatte 15 mit in dieser im Abstand eingeformten Vertiefungen 16 gehalten. Die Vertiefungen 16 weisen am Boden kleine Löcher 17 auf. In der in die Führungsschienen 14 vollständig eingeschobenen Lage der Aufbereitungsplatte 15 liegen die Vertiefungen 16 jeweils über einer Kammer 13a bis 13e. Die zuvor abgetrennten Probensegmente 6a der Gewebeprobe 6 werden in die Vertiefungen 16 gelegt und mit an einem Drehstempel 18 befestigten Drehstempelmesser 19 weiter zerkleinert. Vorzugsweise sind 2 oder mehrere Drehstempelmesser 19 an der Stirnseite des Drehstempels angeordnet. Das Zerkleinern der Probensegmente 6a erfolgt bei leichtem Druck und gegebenenfalls gleichzeitigem Hin- und Herdrehen des Drehstempels 18.

Wie Fig. 2 zeigt, können auch mehrere Drehstempel 18 in einer Halteplatte 20 drehbeweglich und heb- und senkbar gehalten sein. Der Abstand zwischen den Drehstempeln 18 entspricht dem der Vertiefungen 16 in der Aufbereitungsplatte 15.

Nach der weiteren Teilung der Probensegmente 6a stehen deren Teilstücke (Gewebefragmente) und die bei diesem Trennvorgang entstehende Gewebeflüssigkeit, die über die Löcher 17 in den Vertiefungen 16 in die Kammern 13a bis 13e gelangt, für die Zellvermehrung zur Verfügung.

Die in den Figuren 1 bis 5 dargestellten Vorrichtungen zum ortsaufgelösten, sequentiell-parallelen Schneiden und weiteren Aufbereiten der Gewebeprobe bestehen aus einem bis 121°C beständigen, zur Behandlung im Autoklav geeigneten Material, vorzugsweise Teflon, Metall oder Plastik. Für die Schneidmesser wird vorzugsweise Glas verwendet.

Die einzelnen Teilstücke und die jeweilige Gewebeflüssigkeit der ortsaufgelöst hergestellten Probensegmente 6a werden nun separat in einzelne, mit dem gleichen Zellkulturmedium, in dem sich bereits die dem Patienten entnommene Gewebeprobe 6 befand, gefüllte Zellkulturflaschen eingebracht. Das verbleibende Zellkulturmedium, in dem die Gewebeprobe nach der Probenahme aufbewahrt wurde, wird ebenfalls in eine Zellkulturflasche gefüllt. Die Zellkulturflaschen sind jeweils mit einer Biomatrix, hier Collagen oder Polylysin beschichtet. Die Zusammensetzung des hier für die Zellvermehrung in vitro eingesetzten Zellkulturmediums ist in der nachfolgenden Tabelle wiedergegeben:

| **Anorganische Salze** | |
|---|---|
| Ca(NO₃)₂ | 50 mg/L |
| CaCl₂• 2 H₂O | 132 mg/L |
| Kcl | 400 mg/L |
| MgSO₄• 7 H₂O | 150 mg/L |
| Nacl | 6400 mg/L |
| NaHCO₃ | 2100 mg/L |
| Na₂HPO₄ | 400 mg/L |

| **Aminosäuren** | |
|---|---|
| L-Arginin • 4 HCl | 110 mg/L |
| L-Asparagin (freie Base) | 38 mg/L |
| L-Asparaginsäure | 23 mg/L |
| L-Cystin | 31 mg/L |
| L-Glutaminsäure | 25 mg/L |
| L-Glutamin | 296 mg/L |
| Glycin | 13 mg/L |
| L-Histidin (freie Base) | 12 mg/L |
| L-Hydroxyprolin | 10 mg/L |
| L-Isoleucin | 38 mg/L |
| L-Leucin | 38 mg/L |
| L-Lysin **•**HCl | 35 mg/L |
| L-Methionin | 12 mg/L |
| L-Phenylalanin | 16 mg/L |
| L-Prolin | 22 mg/L |
| L-Serin | 26 mg/L |
| L-Threonin | 22 mg/L |
| L-Tryptophan | 5 mg/L |
| L-Tyrosin | 19 mg/L |
| L-Valin | 22 mg/L |
| L-Alanin | 10 mg/L |

| **Vitamine** | |
|---|---|
| Biotin | 0,6 mg/L |
| D-Ca-Pantothenat | 0,7 mg/L |
| Cholinchlorid | 3,5 mg/L |
| Folsäure | 1,0 mg/L |
| i-Inositol | 35,9 mg/L |
| Nicotinamid | 1,0 mg/L |
| Pyridoxal **•** HCl | 1,0 mg/L |
| Riboflavin | 20 µg/L |
| Thiamin **•** HCl | 1,0 mg/L |
| Para-Aminobenzoesäuure | 500 µg/L |
| Vitamin B₁₂ | 5 µg/L |
| Niacin | 25 µg/L |
| Ascorbinsäure | 50 µg/L |
| Folinsäure | 6 µg/L |
| Liponsäure | 21 µg/L |
| Vitamin A (Acetat) | 100 µg/L |
| Pyridoxin **•** HCl | 25 µg/L |
| Niacinamid | 25 µg/L |
| α-Tocopherolphosphat | 10 µg/L |

| **Weitere Komponenten** | |
|---|---|
| D-Glucose | 1750 mg/L |
| Phenolrot | 7 mg/L |
| Glutathion (reduziert) | 0,5 mg/L |
| Na-Pyrovat | 1 mM |
| Epidermaler Wachstumsfaktor (Epidermal Growth Factor, EGF-rekombinant | 250 ng/L |
| Fötales Rinderserum (FBS) | 12,5 % |
| Insulin vom Rind (Lyophilisat) | 8 mg/L (26 U/mg) |

### Antibiotika nach Stand der Technik.

Die mit einer Biomatrix beschichteten Zellkulturflaschen mit dem erfindungsgemäßen Zellkulturmedium und den in diesem befindlichen, nach dem zuvor beschriebenen Aufbereitungsverfahren hergestellten kleinen Gewebefragmenten bzw. der Gewebeflüssigkeit werden anschließend in einen Brutschrank eingebracht und dort bei einer Temperatur zwischen 30°C und 36,5°C, einer Sauerstoffatmosphäre von 0,01 bis 3 %, einer Kohlendioxidatmosphäre von 0,1 bis 5 % und einer Luftfeuchte von 100 % aufbewahrt. Die genaue Temperatur richtet sich nach der Temperatur, die bei der Entnahme der Gewebeprobe gemessen wurde.

Bereits nach 1 bis 12 Stunden erfolgt eine Adhäsion der Tumorzellen an das Biomatrixsubstrat in der Zellkulturflasche. Etwa 24 Stunden nach der Erstetablierung der Kultur wird nach erfolgter Zelladhäsion das in den Zellkulturflaschen befindliche Medium gegen ein frisches Zellkulturmedium, das die gleiche Zusammensetzung wie das erste aufweist, ausgetauscht. Weitere Medienwechsel werden - je nach Präsenz von Kontaminanten - in der ersten Woche durchgeführt. Nachdem die Tumorzellen nach einer Ruhephase etabliert sind und sich vermehren, werden sie in einem von Antibiotika freien Medium gehalten. Daraufhin wird die Massenvermehrung initiiert.

Durch die oben beschriebene frühzeitige Teilung der Gewebeprobe nach 2 bis 24 Stunden in separate Gewebesegmente bzw. noch kleinere Gewebefragmente ist die Wahrscheinlichkeit einer Kontamination und einer Massenvermehrung von Kontaminanten in den Kulturflaschen gering. Dennoch vereinzelt aufgefundene Kulturflaschen mit hoher Kontaminationsbelastung werden verworfen. Bei einer durch technische Fehler bedingten starken Vermehrung von Normalzellen, die zu einem Überwachsen der Tumorzellen führen, kann auch eine Magnetseparation durchgeführt werden, woraufhin unter den oben angegebenen Kulturbedingungen ein selektives Wachstum der malignen Zellen gewährleistet ist.

### Bezugszeichenliste

- 1: Auffangschale
- 1a1 bis 1a10: Unterkammern
- 1b1 bis 1b5: Unterkammern
- 1c1 bis 1c3: Unterkammern
- 1d 1e1 bis 1e10: Unterkammern
- 2: Schneidplatte
- 3: Schneidmesserrahmen
- 4: Schneidrinnen
- 4a: Öffnung in 4
- 5: Auflagefläche
- 6: Gewebeprobe
- 6a: Probensegment
- 7: Führungsschiene
- 8: Einschnitte
- 10: Schneidmesser/Schneiddrähte
- 11: Trennwand
- 12: Trennwand
- 13: Flüssigkeitsauffangschale
- 13a bis 13e: Kammern
- 14: Führungsschienen
- 15: Aufbereitungsplatte
- 16: Vertiefungen
- 17: Löcher
- 18: Drehstempel
- 19: Drehstempelmesser
- 20: Drehstempel-Halteplatte

## Patentansprüche

1. Verfahren zur Kultivierung von Krebszellen aus Humangewebe für molekularbiologische Reihenuntersuchungen, **dadurch gekennzeichnet, daß** eine Gewebeprobe (6) auf der Grundlage ihrer heterogenen Struktur in bezug auf Tumorzellen, Normalzellen und Kontaminanten durch eine sequentiell-parallele Teilung in Scheibensegmente aufgeteilt wird und die einzelnen Gewebeprobensegmente separiert und weiter in Gewebefragmente geteilt werden und die gewonnenen kleinen, separierten Gewebefragmente und Gewebeflüssigkeiten der aufgeteilten Gewebeprobensegmente (6a) separat in mit einer Biomatrix beschichteten und mit einem Zellkulturmedium gefüllten Zellkulturflaschen unter einer Sauerstoffatmosphäre von 0,01 bis 3 % und einer Kohlendioxidatmosphäre von 0,1 - 5 % sowie bei einer Luftfeuchte von 100 % und Temperaturen zwischen 30°C und 36,5°C kultiviert werden, wobei das Zellkulturmedium zusammengesetzt aus
| anorganischen Salzen, nämlich | |
|---|---|
| Ca(NO₃)₂ | 10-100 mg/L |
| CaCl₂, •2H₂O | 80-150 mg/L |
| KCI | 200-1000 mg/L |
| MgSO₄ •7H₂O | 200-700 mg/L |
| NaCl | 3000-10000 mg/L |
| NaHCO₃ | 1500-4000 mg/L |
| Na₂HPO₄ | 100-1000 mg/L; |
| Aminosäuren, nämlich | |
|---|---|
| L-Arginin• 4 HCl | 10-500 mg/L |
| L-Asparagin (freie Base) | 10-500 mg/L |
| L-Asparaginsäure | 10-500 mg/L |
| L-Cystin | 10-500 mg/L |
| L-Glutaminsäure | 10-500 mg/L |
| L-Glutamin | 10-500 mg/L |
| Glycin | 10-500 mg/L |
| L-Histidin (freie Base) | 10-500 mg/L |
| L-Hydroxyprolin | 10-500 mg/L |
| L-Isoleucin | 10-500 mg/L |
| L-Leucin | 10-500 mg/L |
| L-Lysin • HCL | 10-500 mg/L |
| L-Methionin | 10-500 mg/L |
| L-Phenylalanin | 10-500 mg/L |
| L-Prolin | 10-500 mg/L |
| L-Serin | 10-500 mg/L |
| L-Threonin | 10-500 mg/L |
| L-Tryptophan | 10-400 mg/L |
| L-Tyrosin | 10-500 mg/L |
| L-Valin | 10-500 mg/L |
| L-Alanin | 10-300 mg/L |
| Vitaminen, nämlich | |
|---|---|
| Biotin | 0,01-10 mg/L |
| D-Ca-Pantothenat | 0,01-10 mg/L |
| Cholinchlorid | 0,1-50 mg/L |
| Folsäure | 0,01-10 mg/L |
| i-Inositol | 0,1-100 mg/ |
| Nicotinamid | 0,01-10 mg/L |
| Pyridoxal • HCl | 0,01-10 mg/L |
| Riboflavin | 0,1-100 µg/L |
| Thiamin • HCl | 0,1-50 mg/L |
| Para-Aminobenzoesäure | 1-1000 µg/L |
| Vitamin B₁₂ | 1-1000 µg/L |
| Niacin | 1-100 µg/L |
| Ascorbinsäure | 1-5000 µg/L |
| Folinsäure | 1-100 µg/L |
| Liponsäure | 1-100 µg/L |
| Vitamin A (Acetat) | 10-1000 µg/L |
| Pyridoxin • HCl | 1-100 µg/L |
| Niacinamid | 1-100 µg/L |
| α-Tocopherolphosphat | 0-1000 µg/L |
sowie
| | |
|---|---|
| D-Glucose | 100-5000 mg/L |
| Phenolrot | 0,1-1000 mg/L |
| Glutathion (reduziert) | 0,01-10 mg/L |
| Na-Pyruvat | 0,1-50 nM |
| Epidermaler Wachstumsfaktor (Epidermal Growth Factor, EGF) - rekombinant | 1-3000 ng/L |
| Fötales Rinderserum (FBS) Insulin vom Rind (Lyophilisat) | 0,1-50 mg/L |
und Antibiotika.

2. Verfahren nach Anspruch 1, **dadurch gekennzeichnet, daß** die Gewebeprobe (6) zur Anpassung an das Zellkulturmedium in diesem mindestens 2 Stunden, jedoch nicht länger als 24 Stunden bei einer Temperatur zwischen 4°C und 12°C verbleibt.

3. Verfahren nach Anspruch 1, **dadurch gekennzeichnet, daß** eine bestimmte Zeit nach der Erstetablierung der Kultur und erfolgter Zelladhäsion das Zellkulturmedium in der Kulturflasche gegen ein neues, jedoch mit gleicher Zusammensetzung ausgetauscht wird.

4. Verfahren nach Anspruch 3, **dadurch gekennzeichnet, daß** das Zellkulturmedium in Abhängigkeit von der Präsenz von Kontaminanten, wie Bakterien oder Pilze, bei gleichbleibendem oder verringertem Anteil an Antibiotika ausgetauscht wird.

5. Vorrichtung zur Durchführung des Verfahrens nach Anspruch 1, zur aufgeteilten Aufbereitung der Gewebeprobe, **gekennzeichnet durch** einen Schneidapparat zum sequentiell-parallelen Zerteilen der Gewebeprobe (6) in einzelne, voneinander getrennte Gewebesegmente (6a) und der entsprechenden Schnittstelle zugehöriger Gewebeflüssigkeit, bestehend aus einer in Kammern mit Unterkammern (1a1 bis 1a10, 1b1 bis 1b5, 1c1 bis 1c3, 1d und 1e1 bis 1e10) aufgeteilten Auffangschale (1), einer auf dieser lösbar gehaltenen Schneidplatte (2) mit zur Auffangschale (1) hin teilweise offenen Schneidrinnen (4) sowie einem Schneidmesserrahmen (3) für Schneidmesser (10), wobei die Anordnung der Schneidmesser (10) im Schneidmesserrahmen (3) mit der der Schneidrinnen (4) in der Schneidplatte (2) übereinstimmt und jeder Schneidrinne (4) eine sich unter dieser befindenden einzelnen Unterkammer zugeordnet ist; sowie ein Zerkleinerungsgerät zur weiteren Aufbereitung der aufgeteilten Gewebeprobensegmente (6a), die eine in Kammern (13a bis 13e) aufgeteilte Flüssigkeits-Auffangschale (13), eine auf dieser lösbar angebrachte Aufbereitungsplatte (15) mit Vertiefungen (16) sowie Drehstempel (18) mit Drehstempelmessern (19) umfaßt, wobei die Vertiefungen (16) Löcher (17) aufweisen und sich jeweils oberhalb einer Kammer (13a bis 13e) befinden und die Drehstempel (18) den Vertiefungen (16) zugeordnet sind.

6. Vorrichtung nach Anspruch 5, **dadurch gekennzeichnet, daß** die Schneidplatte (2) eine senkrecht zu den Schneidrinnen (4) verlaufende und mittig angeordnete aufgerauhte Auflagefläche (5) zur stabilen Lagerung der Gewebeprobe (6) aufweist.

7. Vorrichtung nach Anspruch 5 und 6, **dadurch gekennzeichnet, daß** die Schneidrinnen (4) im Bereich der Auflagefläche (5) zu der darunterliegenden Unterkammer hin offen sind, so das beim Schneiden gebildete Gewebeflüssigkeit oder Gewebestücke getrennt in der jeweiligen Unterkammer aufgefangen werden.

8. Vorrichtung nach einem der Ansprüche 5 bis 7, **dadurch gekennzeichnet, daß** die Breite der Schneidrinnen (4) größer als die Stärke der Schneidmesser (10) ist.

9. Vorrichtung nach einem der Ansprüche 5 bis 8, **dadurch gekennzeichnet, daß** die Schneidmesser (10) an einer Deckplatte des Schneidmesserrahmens (3) befestigt sind.

10. Vorrichtung nach einem der Ansprüche 5 bis 9, **dadurch gekennzeichnet, daß** die Schneidmesser (10) in dem Schneidmesserrahmen (3) verspannte Schneiddrähte bilden.

11. Vorrichtung nach einem der Ansprüche 5 - 10, **dadurch gekennzeichnet, daß** die Schneidplatte (2) und die Aufbereitungsplatte (15) jeweils in Führungsschienen (7 bzw. 14) an der Auffangschale (1) bzw. der Flüssigkeits-Auffangschale (13) gehalten sind, wobei in den Führungsschienen (7) für die Schneidplatte (2) mit den Schneidrinnen (4) fluchtende Einschnitte (8) ausgebildet sind.

12. Vorrichtung nach einem der Ansprüche 5 - 11, **dadurch gekennzeichnet, daß** die Drehstempel (18) in Bohrungen einer Drehstempel-Halteplatte (20) in senkrechter Richtung bewegbar sowie drehbar angeordnet sind.

## Claims

1. A method of cultivating cancer cells from human tissue for molecular-biological mass screenings, **characterized in, that** a tissue sample (6), based on its heterogeneous structure regarding tumour cells, normal cells and contaminants, is split by a sequential-parallel splitting into disk segments and said single tissue sample segments are separated and further split into tissue fragments and said attained small, separated tissue fragments and tissue fluids of said split tissue sample segments (6a) are cultivated separately in cell culture bottles, coated with a biomatrix and filled with a cell culture medium, in an oxygen atmosphere from 0.01 % to 3% and a carbon dioxide atmosphere from 0.1 % to 5% as well as an air humidity of 100% and temperatures between 30°C and 36.5°C, wherein the cell culture medium is comprised of
| inorganic salts, namely | |
|---|---|
| Ca(NO₃)₂ | 10-100 mg/l |
| CaCl₂ • 2H₂O | 80-150 mg/I |
| KCI | 200-1000 mg/l |
| MgSO₄ • 7H₂O | 200-700 mg/l |
| NaCl | 3000-10000 mg/l |
| NaHCO₃ | 1500-4000 mg/l |
| Na₂HPO₄ | 100-1000 mg/l; |
| amino acids, namely | |
|---|---|
| L-arginine • 4 HCl | 10-500 mg/I |
| L-asparagine (free base) | 10-500 mg/l |
| L-aspartic acid | 10-500 mg/l |
| L-cystine | 10-500 mg/l |
| L-glutamic acid | 10-500 mg/l |
| L-glutamine | 10-500 mg/l |
| Glycine | 10-500 mg/l |
| L-histidine (free base) | 10-500 mg/l |
| L-hydroxyproline | 10-500 mg/l |
| L-isoleucine | 10-500 mg/l |
| L-leucine | 10-500 mg/l |
| L-lysine • HCl | 10-500 mg/l |
| L-methionine | 10-500 mg/l |
| L-phenylalanine | 10-500 mg/l |
| L-proline | 10-500 mg/l |
| L-serine | 10-500 mg/l |
| L-threonine | 10-500 mg/l |
| L-tryptophane | 10-400 mg/l |
| L-tyrosine | 10-500 mg/l |
| L-valine | 10-500 mg/l |
| L-alanine | 10-300 mg/l; |
| vitamins, namely | |
|---|---|
| Biotin | 0.01-10 mg/l |
| D-Ca-pantothenate | 0.01-10 mg/l |
| Choline chloride | 0.1-50 mg/l |
| Folic acid | 0.01-10 mg/l |
| i-Inositol | 0.1-100 mg/l |
| Nicotinamide | 0.01-10 mg/l |
| Pyridoxine • HCl | 0.01-10 mg/l |
| Riboflavin | 0.1-100 µg/l |
| Thiamine • HCl | 0.1-50 mg/l |
| Paraminobenzoic acid | 1-1000 µg/l |
| Vitamin B₁₂ | 1-1000 µg/l |
| Niacin | 1-100 µg/l |
| Ascorbic acid | 1-5000 µg/l |
| Folinic acid | 1-100 µg/l |
| Liponic acid | 1-100 µg/l |
| Vitamin A (acetate) | 10-1000 µg/l |
| Pyridoxine • HCl | 1-100 µg/l |
| Niacinamide | 1-100 µg/l |
| α-Tocopherol phosphate | 0-1000 µg/l |
as well as
| | |
|---|---|
| D-glucose | 100-5000 mg/l |
| Phenol red | 0.1-1000 mg/l |
| Glutathione (reduced) | 0.01-10 mg/l |
| Sodium pyruvate | 0.1-50 nM |
| Epidermal growth factor (EGF) | |
| recombinant | 1-3000 ng/l |
| Fetal bovine serum (FBS) | |
| Bovine insulin (lyophilisate) | 0.1-50 mg/l |
and antibiotics.

2. The method according to claim 1, **characterized in, that** the tissue sample (6) is kept in the cell culture medium for adaptation to said cell culture medium for at least 2 hours but no longer than 24 hours at a temperature between 4°C and 12°C.

3. The method according to claim 1, **characterized in, that** the cell culture medium in the culture bottle is replaced by a fresh medium of the same composition after a defined period of time after the initial establishment of the culture and completed cell adhesion.

4. The method according to claim 3, **characterized in, that** the cell culture medium is replaced with a constant or reduced amount of antibiotics depending on the presence of contaminants such as bacteria or fungi.

5. An apparatus for performing the method according to claim 1 for the split preparation of the tissue sample, **characterized in** a cutting apparatus for sequential-parallel splitting of the tissue sample (6) into single tissue segments (6a) separated from each other and into the respective tissue fluid corresponding to the cutting site, consisting of a collecting pan (1) divided into chambers having sub-chambers (1a1 to 1a10, 1b1 to 1b5, 1c1 to 1c3, 1d, and 1 e1 to 1e10), a cutting plate (2) detachably mounted on top of said collecting pan (1) and having cutting grooves (4) that are partially open towards the collecting pan (1), as well as a cutting blade frame (3) for cutting knives (10), wherein the arrangement of said cutting knives (10) in the cutting blade frame (3) matches said cutting grooves (4) in the cutting plate (2) and each one of said cutting grooves (4) is assigned to a single sub-chamber located beneath it; as well as a grinding device for the further preparation of the split tissue sample segments (6a) comprising a fluid-collecting pan (13) that is divided into chambers (13a to 13e), a detachably mounted preparation plate (15) with recesses (16) on top of it as well as rotatory plungers (18) having plunger knives (19), wherein said recesses (16) are comprising holes (17) and each of being located above a chamber (13a to 13e) and said rotatory plungers (18) are assigned to said recesses (16).

6. The apparatus according to claim 5, **characterized in, that** the cutting plate (2) features a centrally arranged roughened support surface (5) that runs perpendicularly to the cutting groves (4) for a stable bearing of the tissue sample (6).

7. The apparatus according to claims 5 and 6, **characterized in, that** the cutting grooves (4) are open in the area of the support surface (5) towards the sub-chamber located underneath, such that the tissue fluid or tissue pieces resulting from cutting are collected separately in the respective sub-chamber.

8. The apparatus according to one of the claims 5 to 7, **characterized in, that** the width of the cutting grooves (4) is greater than the thickness of the cutting knives (10).

9. The apparatus according to one of the claims 5 to 8, **characterized in, that** the cutting knives (10) are mounted on a covering plate of the cutting blade frame (3).

10. The apparatus according to one of the claims 5 to 9, **characterized in, that** the cutting knives (10) are forming braced cutting wires in the cutting blade frame (3).

11. The apparatus according to one of the claims 5 to 10, **characterized in, that** each of the cutting plate (2) and the preparation plate (15) are maintained in guide rails (7 and 14, respectively) at the collecting pan (1) respectively the fluid-collecting pan (13), wherein recesses (8) flushing with the cutting grooves (4) are formed within the guide rails (7) for the cutting plate (2).

12. The apparatus according to one of the claims 5 to 11, **characterized in, that** the rotatory plungers (18) are arranged in bores of a rotatory plunger supporting plate (20) slidably as well as rotatably in perpendicular direction.

## Revendications

1. Procédé de culture de cellules cancéreuses issues de tissu humain destinées à des examens biomoléculaires en série, **caractérisé en ce qu'**un échantillon de tissu (6) est divisé sur la base de sa structure hétérogène quant aux cellules tumorales, aux cellules normales et aux contaminants par partage séquentiel-parallèle en segments de disque et que les différents segments d'échantillons de tissus sont divisés et ensuite séparés en fragments tissulaires et que les petits fragments tissulaires séparés ainsi obtenus et les liquides tissulaires des segments d'échantillons de tissus (6a) sont cultivés séparément dans des flacons de culture cellulaire tapissés d'une biomatrice et remplis d'un milieu de culture cellulaire sous atmosphère d'oxygène de 0,01 à 3 % et d'une atmosphère de dioxyde de carbone de 0,1 à 5 %, l'humidité de l'air s'élevant à 100 % et les températures étant comprises entre 30 °C et 36,5 °C, le milieu de culture cellulaire étant composé des éléments suivants :
| Sels minéraux, à savoir : | |
|---|---|
| Ca(NO₃)₂ | 10-100 mg/l |
| CaCl₂ . 2H₂O | 80-150 mg/l |
| KCl | 200-1000 mg/l |
| MgSO₄.7H₂O | 200-700 mg/l |
| NaCl | 3000-10000 mg/l |
| NaHCO₃ | 1500-4000 mg/l |
| Na₂HPO₄ | 100-1000 mg/l |
| Acides aminés, à savoir : | |
|---|---|
| L-arginine . 4 HCl | 10-500 mg/l |
| L-asparagine (base libre) | 10-500 mg/l |
| Acide L-asparaginique | 10-500 mg/l |
| L-cystine | 10-500 mg/l |
| Acide L-glutamique | 10-500 mg/l |
| L-glutamine | 10-500 mg/l |
| Glycine | 10-500 mg/l |
| L-histidine (base libre) | 10-500 mg/l |
| L-hydroxyproline | 10-500 mg/l |
| L-isoleucine | 10-500 mg/l |
| L-leucine | 10-500 mg/l |
| L-lysine . HCl | 10-500 mg/l |
| L-méthionine | 10-500 mg/l |
| L-phénylalanine | 10-500 mg/l |
| L-proline | 10-500 mg/l |
| L-sérine | 10-500 mg/l |
| L-thréonine | 10-500 mg/l |
| L-tryptophane | 10-400 mg/l |
| L-tyrosine | 10-500 mg/l |
| L-valine | 10-500 mg/l |
| L-alanine | 10-300 mg/l |
| Vitamines, à savoir : | |
|---|---|
| Biotine | 0,01-10 mg/l |
| D-Ca-panthothénate | 0,01-10 mg/l |
| Chlorure de choline | 0,1-50 mg/l |
| Acide folique | 0,01-10 mg/l |
| i-Inositol | 0,1-100 mg/l |
| Nicotinamide | 0,01-10 mg/l |
| Pyridoxal . HCl | 0,01-10 mg/l |
| Riboflavine | 0,1-100 µg/l |
| Thiamine . HCl | 0,1-50 mg/l |
| Acide para-aminobenzoïque | 1-1000 µg/l |
| Vitamine B₁₂ | 1-1000 µg/l |
| Niacine | 1-100 µg/l |
| Acide ascorbique | 1-5000 µg/l |
| Acide folinique | 1-100 µg/l |
| Acide lipoïque | 1-100 µg/l |
| Vitamine A (acétate) | 10-1000 µg/l |
| Pyridoxine . HCl | 1-100 µg/l |
| Niacinamide | 1-100 µg/l |
| α-Tocophérolphosphate | 0-1000 µg/l |
et
| | |
|---|---|
| D-glucose | 100-5000 mg/l |
| Rouge de phénol | 0,1-1000 mg/l |
| Glutathion (réduit) | 0,01-10 mg/l |
| Na-pyruvate | 0,1-50 nM |
| Facteur de croissance épidermique (Epidermal Growth Factor, EGF) - recombinant | 1-3000 ng/l |
| Sérum bovin foetal (FBS) Insuline bovine (lyophilisat) | 0,1-50 mg/l |
et des antibiotiques.

2. Procédé selon la revendication 1, **caractérisé en ce que** l'échantillon tissulaire (6) reste pour s'y adapter dans le milieu de culture cellulaire au moins 2 heures, toutefois sans excéder 24 heures à une température comprise entre 4 °C et 12 °C.

3. Procédé selon la revendication 1, **caractérisé en ce qu'**un certain temps après la mise en route de la culture et après adhésion des cellules, le milieu de culture cellulaire est échangé contre un nouveau milieu de composition identique.

4. Procédé selon la revendication 3, **caractérisé en ce que** le milieu de culture cellulaire est échangé en fonction de la présence de contaminants, comme des bactéries ou des champignons, la fraction d'antibiotiques n'étant pas modifiée ou étant réduite.

5. Dispositif destiné à la mise en oeuvre du procédé selon la revendication 1, pour la préparation de l'échantillon tissulaire **caractérisé, par** un appareil de coupe destiné au partage séquentiel parallèle de l'échantillon tissulaire (6) en segments de tissu (6a) individuels, séparés les uns des autres et, liquide tissulaire correspondant à la zone de coupe, composé d'un récipient (1) divisé en compartiments et sous-compartiments (1a1 à 1a10, 1b1 à 1b5, 1c1 à 1c3, 1d et 1e1 à 1e10), d'une plaque de coupe (2) montée sur celui-ci de façon amovible, présentant des rainures de coupe (4) partiellement découvertes dans le prolongement jusqu'au récipient (1), ainsi qu'un cadre destiné aux lames de coupe (3) recevant les lames de coupe (10), la disposition des lames de coupe (10) dans le cadre des lames de coupe (3) correspondant à celle des rainures de coupe (4) dans la plaque de coupe (2) et à chaque rainure de coupe (4) étant associé un sous-compartiment individuel placé en dessous; ainsi qu'un broyeur permettant de poursuivre la préparation des segments d'échantillons tissulaires partagés (6a), qui englobe un réceptacle de liquide (13) divisé en compartiments (13a à 13e), une plaque de préparation montée sur celui-ci de façon amovible sur ce réceptacle (15) comportant des cupules (16) ainsi que des poinçons rotatifs (18) avec mesureurs de poinçons rotatifs (19), les cupules (16) présentant des trous (17) et étant placées respectivement au-dessus d'un compartiment (13a à 13e) et les poinçons rotatifs (18) étant associés aux cupules (16).

6. Dispositif selon la revendication 5, **caractérisé en ce que** la plaque de coupe (2) présente une surface d'appui (5) centrale rugueuse s'étendant en direction perpendiculaire aux rainures de coupe (4) et disposée de façon centrée permettant une position stable de l'échantillon tissulaire (6).

7. Dispositif selon les revendications 5 et 6, **caractérisé en ce que** les rainures de coupe (4) au niveau de la surface d'appui (5) sont ouvertes en direction de compartiments sous-jacents, de façon que le liquide tissulaire ou les fragments tissulaires qui se forment lors de la coupe soient recueillis séparément dans le sous-compartiment respectif.

8. Dispositif selon les revendications 5 à 7, **caractérisé en ce que** la largeur des rainures de coupe (4) est supérieure à l'épaisseur des lames (10).

9. Dispositif selon une des revendications 5 à 8, **caractérisé en ce que** les lames (10) sont fixées sur une plaque de couverture du cadre des lames (3).

10. Dispositif selon une des revendications 5 à 9, **caractérisé en ce que** les lames (10) forment des fils de coupe tendus dans le cadre des lames (3).

11. Dispositif selon une des revendications 5 à 10, **caractérisé en ce que** la plaque de coupe (2) et la plaque de préparation (15) sont respectivement maintenues dans des rails de guidage (7 ou 14) contre le récipient (1) ou le réceptacle destiné aux liquides (13), les rails de guidage (7) pour la plaque de coupe (2) portant des entailles (8) alignées avec les rainures de coupe (4).

12. Dispositif selon une des revendications 5 à 11, **caractérisé en ce que** les poinçons rotatifs (18) sont placés dans les perçages d'une plaque portant les poinçons rotatifs (20) de manière à être déplaçable en position verticale et susceptible de tourner.
